# EUROPÄISCHE PATENTANMELDUNG

(11) **EP 1 764 363 A2**
(43) Veröffentlichungstag der Anmeldung: **21.03.2007**
(21) Anmeldenummer: 06019204.4
(22) Anmeldetag: 13.09.2006
(51) Int. Cl.: C07D 307/40, A61K 31/015, C07C 15/12

(54) **Substituierte Acetophenonderivate**

(30) Priorität: 15.09.2005 DE 102005044156
(71) Anmelder: Riemser Arzneimittel AG, 17493 Greifswald - Insel Riems (DE)
(72) Erfinder: Quincoces Suarez, José Augustin, Bairro Santa Cecilia, 01226-030Sao Paulo (BR); Peseke, Klaus, 18107 Elmenhorst/Lichtenhagen (DM); Molina Ruiz, Reinaldo, 18107 Elmenhorst/Lichtenhagen (DE)
(74) Vertreter: Vossius & Partner

(57) **Zusammenfassung**

Die Erfindung bezieht sich auf substituierte Acetophenonderivate die antiproliferative, radikalfangende, antiseptische, antimikrobielle und/oder immunstimmulierende bzw. immunmodulierende Eigenschaften aufweisen, Arzneimittel die diese enthalten, sowie auf ein Verfahren zu deren Herstellung.

## Beschreibung

Die Erfindung bezieht sich auf neue substituierte Acetophenonderivate vom Typ der Chalkone, Chromane und Knoevenagelprodukte die antiproliferative, radikalfangende, antiseptische, antimikrobielle, und/oder immunstimulierende bzw. immunmodulierende Eigenschaften aufweisen, auf Arzneimittel die diese enthalten, deren Verwendung zur Herstellung von Arzneimitteln sowie auf Verfahren zu deren Herstellung.

Matsushima et al., Bull. Chem. Soc. Japan, 1980, 53, 2938-42; Alcantara et al., Tetrahedron Letters, 1987, 28(14), 1515-1518;
Gupta et. al., J. of Indian Council of Chemists, 1988, 4(1), 47-50; Krishnamurty et al., Indian J. of Chemistry, 1989, 28B, 279-281, Maruyama et. al., Tetrahedron Letters, 1989, 30(31), 4145-8; Lu et al., Chinese Science Bulletin, 1993, 38(19), 1607-11; Climent et al. (J. of Catalysis, 1995, 151(1), 60-6) beschreiben die Herstellung von 1-(2-Hydroxyphenyl)-3-(4-methoxyphenyl)propenon.

Forestier et al. beschreiben die Anwendung von 1-(2-Hydroxyphenyl)-3-(4-methoxyphenyl)propenon als Schutzmittel für Haut und Haare gegen die Wirkung von UV-Licht ( DE 3742690 A1, 1988).
Eigenschaften des Photowiderstandes von 1-(2-Hydroxyphenyl)-3-(4-methoxyphenyl)propenon sind Gegendtand von JP 03044642 A2 1991 02 26.
Die UV-absorbierenden Eigenschaften von 1-(2-Hydroxyphenyl)-3-(4-methoxyphenyl)propenon für Anwendungen in der kosmetischen Industrie sind Gegenstand von J P 63166848 A2 1988 07 11.

Weder eine antitumorale Wirkung dieser Verbindung noch eine antibakterielle Wirkung von 30 Verbindungen aus der Klasse der Chalcone, darunter auch die von 1-(2-Hydroxy-phenyl)-3-(4-methoxy-phenyl)-propenon konnte bisher nachgewiesen werden (Bioorganic & Medicinal Chemistry Letters, 2002, 12, 2685-2687, Revista Argentina de Microbiologia, 1985, 17, 27-32, Acta Pharmaceutica Hungarica, 1976, 46, 49-56).

Die erfindungsgemässen substituierten Chalcone der allgemeinen Formel **III**, die Chroman-4-one der allgemeinen Formel **IV** und die Knoevenagelprodukte der allgemeinen Formel **VI** wurden bisher noch nicht hergestellt und ihre antiproliferativen, radikalfänger- und antiseptischen und/oder immunstimulierenden bzw. immunmodulierenden Eigenschaften sind bisher noch nicht bekannt.

Die vorliegende Erfindung stellt somit substituierte Acetophenoderivate vom Typ der Chalkone (III), Chromane (IV) und Knoevenagelprodukte (VI) die antiproliferative, radikalfangende, antiseptische, antimikrobielle, und/oder immunstimulierende bzw. immunmodulierende Eigenschaften aufweisen, Arzneimittel die diese enthalten, deren Verwendung zur Herstellung von Arzneimitteln sowie auf Verfahren zu ihrer Herstellung bereit.

Die neuen erfindungsgemässen Chalkonderivate haben die allgemeine Formel **III** 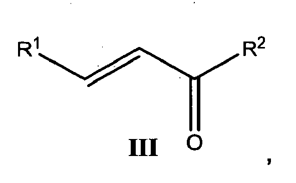 wobei
R¹ einen 5-Hydroxymethylfuran-2-ylrest, einen 4-Methoxyphenylrest, einen 4-Hydroxy-3-methoxyphenylrest, Furan-2-ylrest oder einen 3-Methoxy-4-(3-methylbut-2-enyloxy)phenylrest darstellt und R² einen 4-Chlorphenylrest, einen 2-Hydroxyphenylrest, 2-Hydroxy-5-(3-methylbut-2-enyl)phenylrest, 2,4-Dihydroxyphenylrest, 2,4,6-Trihydroxyphenylrest, 4-Hydroxyphenylrest, 2,2-Dimethyl-2H-chromen-6-ylrest, oder einen 2-(3-Methylbut-2-enyloxy)phenylrest darstellt,
mit der Ausnahme von 1-(2-Hydroxyphenyl)-3-(4-methoxyphenyl)propenon und 3-(4-Hydroxy-3-methoxyphenyl)- 1-(2-hydroxyphenyl)propenon.

Die neuen erfindungsgemässen Chromanderivate haben die allgemeine Formel IV 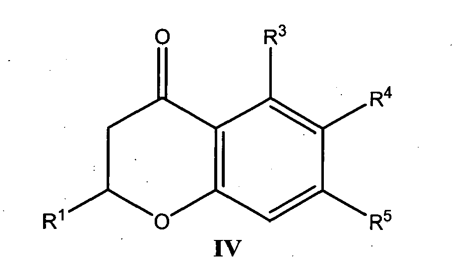 wobei
R¹ einen 5-Hydroxymethylfuran-2-ylrest, einen 4-Methoxyphenylrest, einen 4-Hydroxy-3-methoxyphenylrest, 3-Methoxy-4-(3-methylbut-2-enyloxy)phenylrest, oder einen Furan-2-ylrest darstellt und
R³, R⁴ und R⁵ unabhängig voneinander ein Wasserstoffatom, einen 5-Hydroxymethylfuran-2-ylrest, einen 4-Methoxyphenylrest, einen 4-Hydroxy-3-methoxyphenylrest oder einen 3-Methoxy-4-(3-methylbut-2-enyloxy)phenylrest darstellen, mit der Massgabe, dass mindestens ein Rest von R³, R⁴ und R⁵ ein Wasserstoffatom ist.

Bevorzugt stellt R¹ einen 5-Hydroxymethylfuran-2-ylrest, einen 4-Methoxyphenylrest, einen 4-Hydroxy-3-methoxyphenylrest oder einen 3-Methoxy-4-(3-methylbut-2-enyloxy)phenylrest dar und R³, R⁴ und R⁵ sind jeweils ein Wasserstoffatom.

Die neuen erfindungsgemässen Knoevenagelprodukt haben die allgemeine Formel **VI** 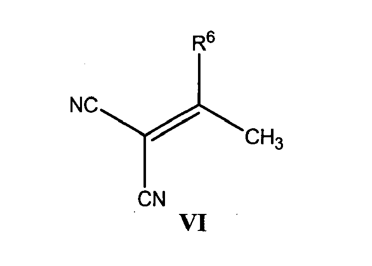 wobei R⁶ einen 2-(3-Methylbut-2-enyloxy)phenylrest, einen 4-(3-Methylbut-2-enyloxy)phenylrest oder einen 4-Hydroxphenylrest darstellt.

Erfindungsgemäß können substituierte Chalcone der allgemeinen Formel **III,** in der R¹ und R² für einen substituierten Phenyl- bzw. Hetarylrest stehen, durch Umsetzung der substituierten Aldehyde der allgemeinen Formel **I,** in der R¹ wie oben definiert ist, mit substituierten Ketonen der allgemeinen Formel **II,** in der R² wie oben definiert ist, in Gegenwart einer Base zur Bildung der entsprechende substituierten Chalcon-Verbindungen unter den Arbeitsbedingungen AAV1, AAV2 bzw. AAV3 umgesetzt werden.
Die Verbindungen der allgemeinen Formel **III** , in der R¹ für einen substituierten Phenyl-, bzw. Hetarylrest steht, werden in Gegenwart einer Base unter Bildung von substituierten Chroman-4-on **IV** hergestellt, in dem R¹ für einen substituierten Phenyl-, Aralkyl- bzw. Hetarylrest steht.
Die Knoevenagelprodukte **VI,** in der R⁶ für einen substituierten Phenyl-, bzw. Hetarylrest steht, werden in Gegenwart einer Mischung bestehend aus Essigsäure, Ammoniumacetat und Toluol nach der Variante der Cope-Synthese hergestellt.

Erfindungsgemäss können die substituierten Chalkonderivate der allgemeinen Formel **III:** 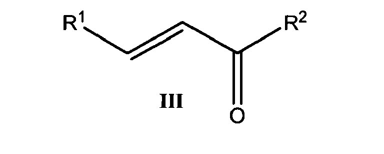 wobei R¹ einen 5-Hydroxymethylfuran-2-ylrest, einen 4-Methoxyphenylrest, einen 4-Hydroxy-3-methoxyphenylrest, Furan-2-ylrest oder einen 3-Methoxy-4-(3-methylbut-2-enyloxy)phenylrest darstellt und R² einen 4-Chlorphenylrest, einen 2-Hydroxyphenylrest, 2-Hydroxy-5-(3-methylbut-2-enyl)phenylrest, 2,4-Dihydroxyphenylrest, 2,4,6-Trihydroxyphenylrest, 4-Hydroxyphenylrest, 2,2-Dimethyl-2H-chromen-6-ylrest oder einen 2-(3-Methylbut-2-enyloxy)phenylrest darstellt dadurch hergestellt werden indem substituierte Aldehyde der allgemeinen Formel **I:** 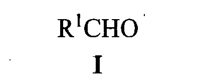 wobei R¹ wie vorstehend definiert ist, in Gegenwart einer Base in einem organischen Lösungsmittel bei einer Reaktionstemperatur im Bereich von 20°C bis 70°C mit einem substituierten Acetophenonderivat der Formel **II:** 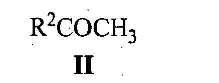 wobei R² wie vorstehend definiert ist, umgesetzt werden, um ein Chalkonderivat der allgemeinen Formel **III** zu erhalten.

Bevorzugt ist die Base Kaliumhydroxid, Bariumhydroxid oder Piperidin.

Ferner ist es bevorzugt, dass das organische Lösungsmittel gegebenenfalls wasserfreies Ethanol oder Methanol ist.

Erfindungsgemäss können die Chromanderivate der allgemeinen Formel **IV** 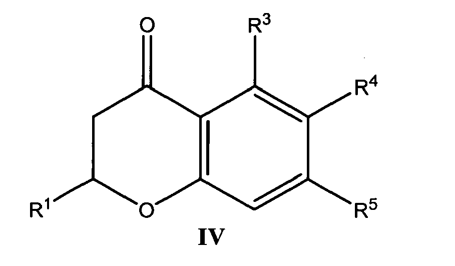 wobei R¹ wie oben definiert ist und
R³, R⁴ und R⁵ unabhängig voneinander ein Wasserstoffatom, einen 5-Hydroxymethylfuran-2-ylrest, einen 4-Methoxyphenylrest, einen 4-Hydroxy-3-methoxyphenylrest oder einen 3-Methoxy-4-(3-methylbut-2-enyloxy)phenylrest darstellen, mit der Massgabe, dass mindestens ein Rest von R³, R⁴ und R⁵ ein Wasserstoffatom ist, hergestellt werden indem ein Chalkon-Derivat der allgemeinen Formel **IIIa** 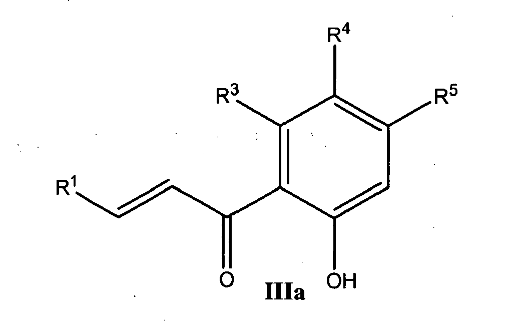 wobei R¹, R³, R⁴ und R⁵ wie vorstehend definiert sind, in Gegenwart einer Base umgesetzt wird, um das substituierte Chroman-4-on-Derivat IV zu erhalten.

Hierbei ist es bevorzugt, dass R¹ einen 5-Hydroxymethylfuran-2-ylrest, einen 4-Methoxyphenylrest, einen 4-Hydroxy-3-methoxyphenylrest oder einen 3-Methoxy-4-(3-methylbut-2-enyloxy)phenylrest darstellt,
und R³, R⁴ und R⁵ jeweils ein Wasserstoffatom sind.

Ferner ist es bevorzugt, dass die hierbei verwendete Base Natriumacetat-Trihydrat ist und/oder das organische Lösungsmittel Ethanol ist.

Weiterhin wird bei dem obengenannte Verfahren die Umsetzung bevorzugt durch Erwärmen und unter Rückfluss durchgeführt wird.

Erfindungsgemäss können die Knoevenagelprodukte der allgemeinen Formel **VI** 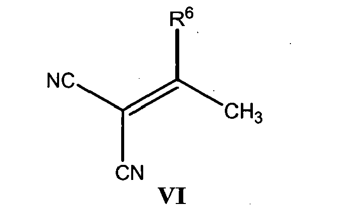 wobei R⁶ einen 2-(3-Methylbut-2-enyloxy)phenylrest, einen 4-(3-Methylbut-2-enyloxy)phenylrest oder einen 4-Hydroxphenylrest darstellt, dadurch hergestellt werden, dass ein Keton der allgemeinen Formel V 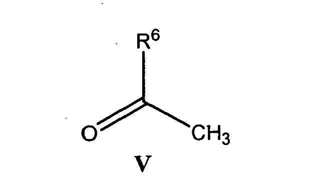 wobei R⁶ wie vorstehend definiert ist, in Gegenwart von Malononitril und einer Mischung aus Ammoniumacetat, Essigsäure und einem organischen Lösungsmittel umgesetzt wird.

Bei diesem Verfahren wird die Umsetzung bevorzugt durch Erwärmen und unter Rückfluss durchgeführt wird.

Die Reaktionsdauer beträgt hierbei bevorzugt 8 bis 10 Stunden.

Ferner ist es bevorzugt, dass das verwendete organische Lösungsmittel Toluol ist.

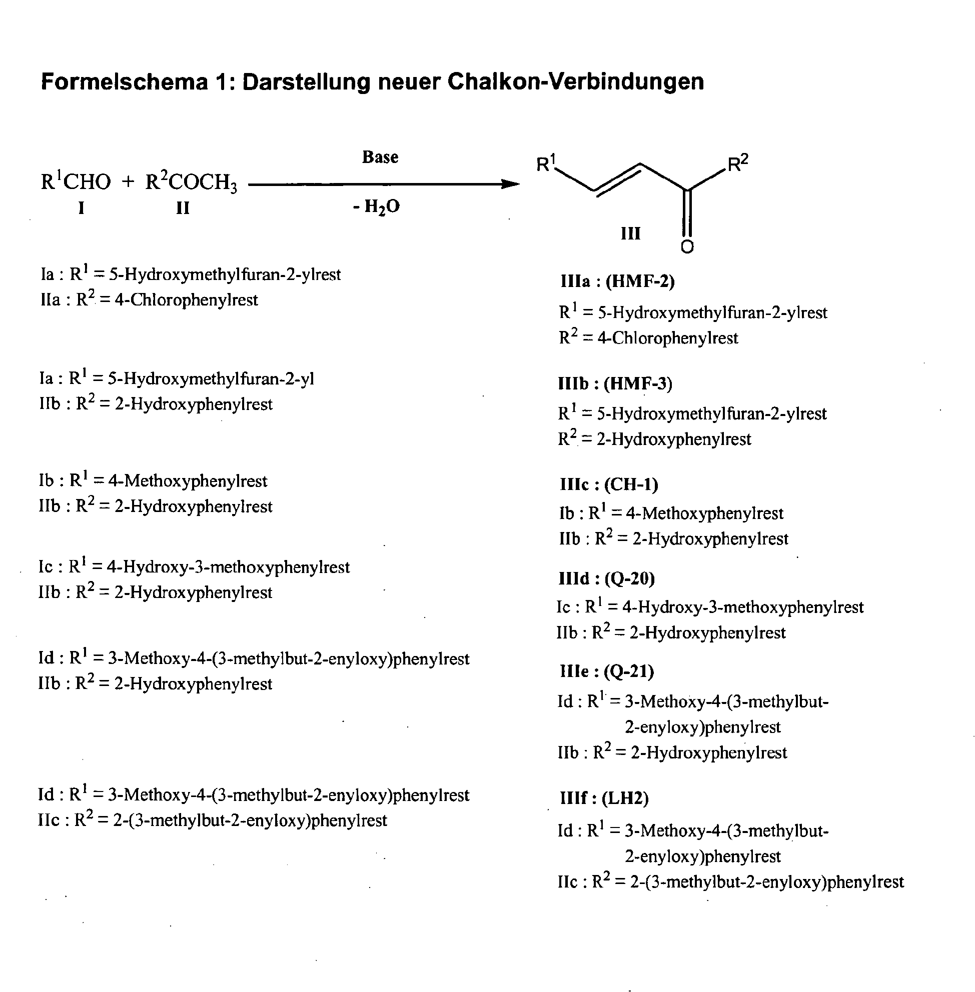

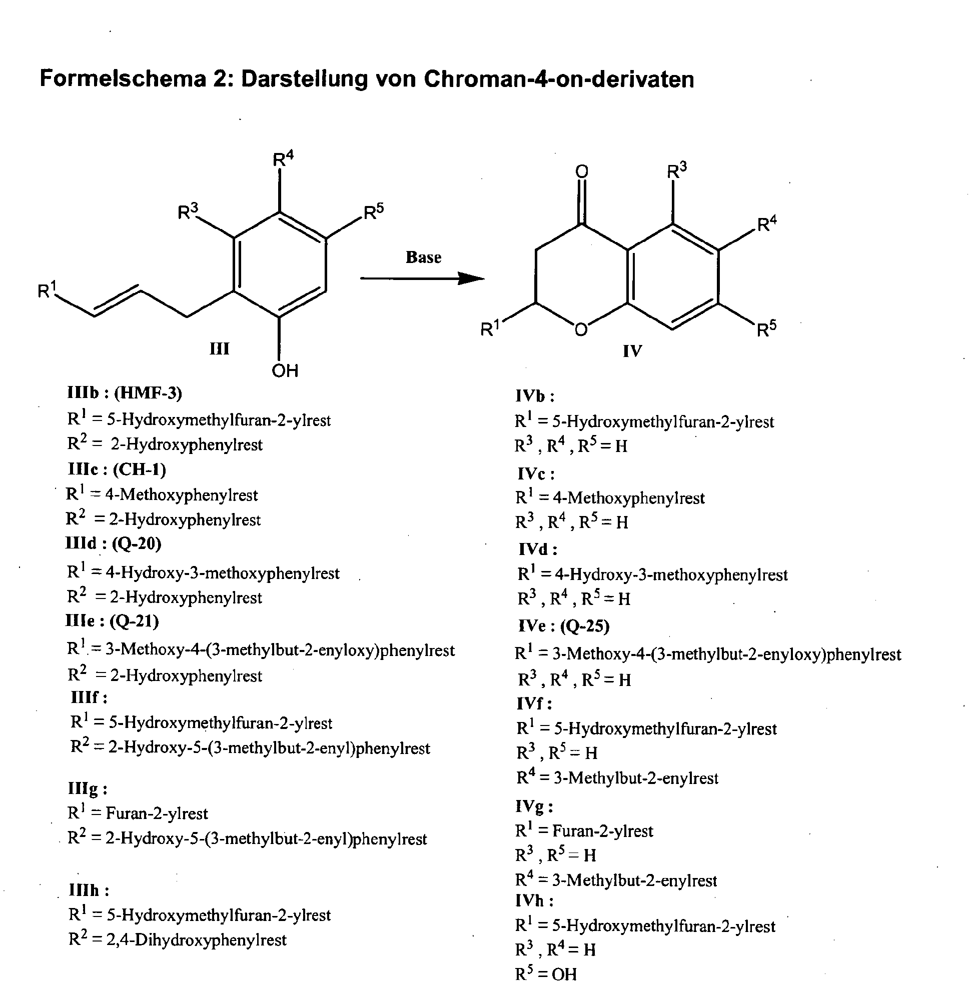

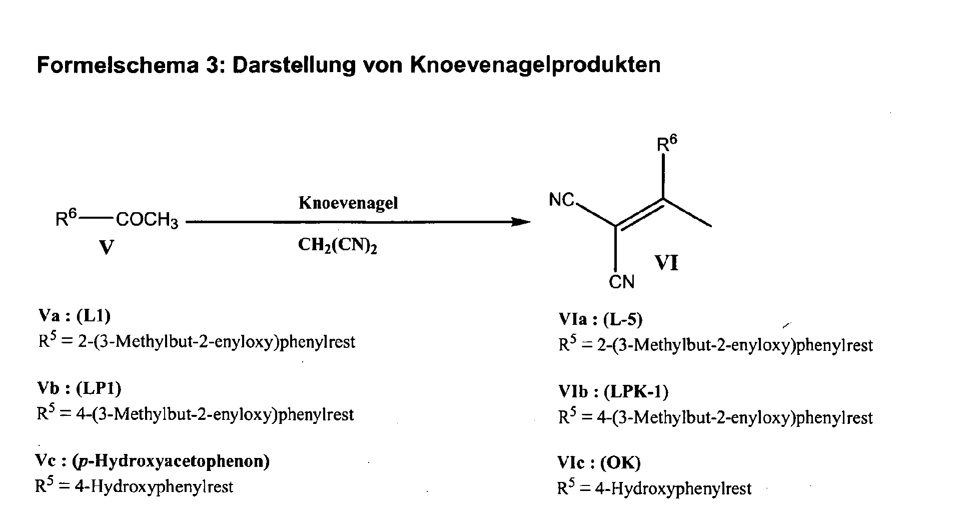

Sowohl die nach den vorstehenden Verfahren hergestellten Verbindungen der Formeln III, IV als auch VI zeigen bisher unbekannte antiproliferative, radikalfangende, antiseptische und antimikrobielle Eigenschaften auf.

Bevorzugte Verbindungen der vorliegenden Erfindung und zur Verwendung gemäss der Erfindung sind die nachstehenden Verbindungen der Formel III:
1-(4-Chlorphenyl)-3-(5-hydroxymethylfuran-2-yl)propenon,
3-(5-Hydroxymethylfuran-2-yl)-1-(2-hydroxyphenyl)propenon,
1-(2-Hydroxyphenyl)-3-[3-methoxy-4-(3-methylbut-2-enyloxy)phenyl]propenon und
3-[3-Methoxy-4-(3-methylbut-2-enyloxy)phenyl]-1-[2-(3-methylbut-2-enyloxy)phenyl]propenon und
1-(2-Hydroxyphenyl)-3-(4-methoxyphenyl)propenon und 3-(4-Hydroxy-3-methoxyphenyl)- 1-(2-hydroxyphenyl)propenon, wobei die 2 letzteren schon als Stoff bekannt sind;
der Formel IV:
   2-[3-Methoxy-4-(3-methylbut-2-enyloxy)phenyl]chroman-4-on,
   2-(5-Hydroxymethylfuran-2-yl)chroman-4-on,
   2-(4-Methoxyphenyl]chroman-4-on und
   2-(4-Hydroxy-3-methoxyphenyl)chroman-4-on;
und der Formel VI:
   2-{1-[2-(3-Methylbut-2-enyloxy)phenyl]ethyliden}malononitril,
   2-[1-(4-Hydroxyphenyl)ethyliden]malononitril und
   2-{1-[4-(3-Methyl-but-2-enyloxy)phenyl]ethyliden}malononitril.

Die Chalkone, Chromane und Knoevenagelprodukte der Formeln III, IV und VI wurden, mit Ausnahme von Q-20 und CH-1, noch nicht beschrieben und stellen somit einen weiteren Aspekt der Erfindung dar.

### Allgemeine Arbeitsvorschriften (AAV) zur Synthese der Chalkone

Die Synthese der Chalkon-Verbindungen erfolgte nach dem Darstellungsmethode von Claisen-Schmidt.

### AA V 1 Claisen-Schmidt-Kondensation mit Kaliumhydroxyd.

0,01 mol des entsprechenden Aldehyds und 0,01 mol des entsprechenden Ketons werden in Gegenwart von Ethanol mit einer 15%igen Kaliumhydroxydlösung unter Rühren umgesetzt. Die Reaktionsmischung wird danach weiterhin ca. 4 Stunden gerührt. Anschließend wird die Mischung mit einer wäßrigen Essigsäure (10%) neutralisiert und gleich danach mit Dichlormethan dreimal extrahiert. Die organische Phase wird mit Wasser gewaschen. Die Trocknung der organischen Phase erfolgt über Natriumsulfat. Nach Filtration wird die Lösung unter vermindertem Druck eingeengt und der Rückstand wird umkristallisiert oder chromatographisch aufgearbeitet.

*AA V 2 Claisen-Schmidt-Kondensation mit Bariumhydroxid.*

0,01 mol des entsprechenden Aldehyds und 0,01 mol des entsprechenden Ketons werden in Gegenwart von Methanol bzw. Ethanol mit 0,01 mol Bariumhydroxyd unter Rühren umgesetzt. Die Reaktionsmischung wird danach weiterhin ca. 8 Stunden gerührt. Nach Abschluß der Reaktion wird das Lösungsmittel unter Vakuum entfernt.
Anschließend wird die Mischung auf 50 ml Eiswasser gegossen und mit einer wäßrigen HCI-Lösung neutralisiert und gleich danach mit Chloroform dreimal extrahiert. Die organische Phase wird mit einer gesättigten Natriumchloridlösung und danach mit Wasser gewaschen. Die Trocknung der organischen Phase erfolgt über Natriumsulfat. Nach Filtration wird die Lösung unter vermindertem Druck eingeengt und der Rückstand wird umkristallisiert oder chromatographisch aufgearbeitet.

*AAV 3 Claisen-Schmidt-Kondensation mit Piperidin.*

0,01 mol des entsprechenden Aldehyds und 0,01 mol des entsprechenden Ketons werden in Gegenwart von absolutem Ethanol mit 2 ml absolutem Piperidin unter Rühren umgesetzt. Man erhitzt die Reaktionsmischung auf 60-70 °C für die Dauer von ca. 8 Stunden weiterhin unter Rühren.
Nach Beendigung der Reaktion wird die Mischung auf 50 ml Eiswasser eingegossen und mit einer wäßrigen HCl-Lösung neutralisiert. Das ausgefallene Produkt wird mit Aktivkohle in Ethanol 15 Minuten behandelt und unmittelbar danach abfiltriert. Das Filtrat wird mit etwas Kieselgel 60 gemischt und unter Vakuum eingeengt. Der Rückstand wird chromatographisch aufgearbeitet.

### Allgemeine Arbeitsvorschrift zur Synthese der Knoevenagelprodukte (AAV 4)

Man erhitzt eine Lösung von 0,01 mol des entsprechenden Ketons, 0,01 mol der entsprechenden CH-aciden Verbindung und 3,5 g Ammoniumacetat in einem Lösungsmittel von 40 ml Toluen und 10 g Essigsäure unter Rückfluss am Wasserabscheider. Nach Beendigung der Reaktion (ca. 8 Stunden) wird das Lösungsmittel am Rotationsverdammpfer eingeengt. Der Rückstand wird in Ethylether aufgenommen, mit einer Lösung Natriumbicarbonat und dann mit Wasser gewaschen. Man trocknet mit Natriumsulfat, ent die Lösung im Vakuum zur Trockne ein und kristallisiert den Rückstand aus den ensprechenden Lösungsmittel um.

### Ausführungsbeispiele:

### 1.1 Synthese von 1-(4-Chlorophenyl)-3-(5-hydroxymethylfuran-2-yl)propenon (HMF-2)

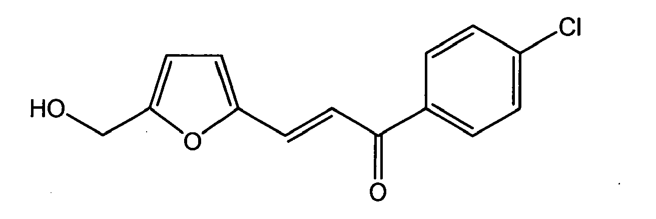

0,01mol (1,26 g) 5-Hydroxymethylfurfural werden mit 0,01mol (13ml) 4-Chloroacetophenon in 200 ml Ethanol in Gegenwart von Kaliumhydroxyd entsprechend der Arbeitsvorschrift AA1 umgesetzt. Das Produkt wird aus einem Ethanol / Ether-Gemisch umkristallisiert.
Summenformel: C₁₄H₁₁ClO₃; Molmasse: 262.5
Gemessene Werte für
Fp: 81-83 °C
Ausbeute: 75 %.
Charakterisierende spektrometische Daten:
**¹H-NMR (CDCl₃):** 2.49 (s, 1H, OH); 4.62 (s, 2H, H-4, CH₂); 6.39 (q, 1H, H-4'), 6.62 (d, 1 H, H-3'); 7.90 (d, 1 H, H-3); 7.75 (d, 2H, H-6"/H-2"); 7.56 (d, 1 H, H-2); 7.46 (d, 2H, H-3"/H-5").
**¹³C-NMR (CDCl₃):** 188.56 (CO); 157.16 (C-5'); 151.37 (C-2'); 139.30 (C-4"); 136.32 (C-1"); 131.02(C-3); 130.30 (C-2"/C-6"); 129.86 (C-3"/C-5"); 118.42 (C-2); 117.84 ( C-3'); 110.73 (C-4'); 57.62 (C-4, CH₂OH).
**NMR-DEPT (CDCl₃):** 131.02(C-3); 130.30 (C-2"/C-6"); 129.86 (C-3"/C-5"); 118.42 (C-2); 117.84 ( C-3'); 110.73 (C-4'); 57.62 (C-4, CH₂OH).
**IR (aufgenommen als Film):** 3267 (OH), 3090 (=CH ), 2866 (CH₂), 1652 (C=O), 1582 (C=C) cm⁻¹.
**MS (70 eV):** 262 M^{+.} e (264 M^{+.} +2) im Verhältnis 3:1; 227 (M^{+.} -35(Cl).

### 1.2 Synthese von 3-(5-Hydroxymethylfuran-2-yl)-1-(2-hydroxyphenyl)-propenon (HMF-3)

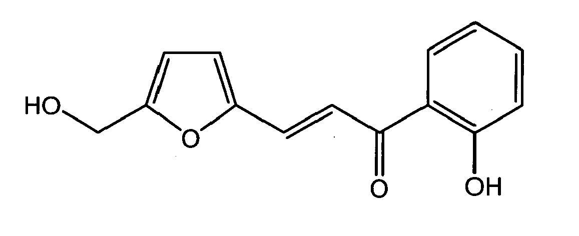

### Variante 1:

Zu einer Mischung, bestehend aus 0,01mol (1,26 g) 5-Hydroxymethylfurfural, 0,01mol (12 ml) 2-Hydroxyacetophenon und 200 ml Ethanol, werden 1,7 ml KOH (15%) hinzugefügt und entsprechend entsprechend der Arbeitsvorschrift AA1 umgesetzt.
Zur chromatographischen Aufarbeitung wird als Eluent eine Mischung aus Toluol und Ethylacetat im Verhältnis 5:1 verwendet. Das Produkt kristallisiert nach dem Einengen des Lösungsmittels analysenrein.
Summenformel: C₁₄H₁₂O₄ ; Molmasse: 244
Gemessene Werte für
Fp: 97-99 °C
Ausbeute: 72 %

### Variante 2:

Zu einer Mischung, bestehend aus 0,01mol 1,26 g 5-Hydroxymethylfurfural, 0,01 mol (12 ml) 2-Hydroxyacetophenon und 2001 Methanol werden 12,5 mmol (4g) Bariumhydroxid hinzugefügt und entsprechend der Arbeitsvorschrift AA2 umgesetzt.
Zur chromatographischen Aufarbeitung wird als Eluent eine Mischung aus Toluol und Ethylacetat im Verhältnis 5:1 verwendet. Das Produkt kristallisiert nach dem Einengen des Lösungsmittel analysenrein.
Gemessene Werte für die
Ausbeute: 60 %

| Elementaranalyse: | | |
|---|---|---|
| Berechnet für die Summenformel C₁₄H₁₂O₄: | 68,85 % C ; | 4,92 % H |
| Gefunden: | 68,81 % C ; | 4,89 % H |

Charakterisierende spektrometrische Daten:
**¹H-NMR (CDCl₃):** 2.30 (s, 1H, OH); 4.70 (s, 2H, H-4, CH₂); 6.45 (q, 1H, H-4'), 6.70 (d, 1 H, H-3'); 6.92 (m, 1 H, H-3" ); 7.01 (m,1 H, H-5" ); 7.37 (m,1H, H-4"); 7.64 (m, 1 H, H-6" ); 7.56 (d, 1 H, H-2); 7.95 (d, 1 H, H-3).
**¹³C-NMR (CDCl₃):** 193.30 (CO); 163.49 (C-2"); 157.32 (C-5'); 151.43 (C-2'); 136.37 (C-3); 131.02(C-4" ); 129.71 (C-6"); 120.03 (C-1"); 118.91 (C-5" ); 118.52 ( C-2); 118.24 (C-3"); 117.49 (C-3'); 110.87 (C-4'); 57.67 (C-4, CH₂OH).
**NMR-DEPT (CDCl₃):** 136.58 (C-3); 131.21 (C-4" ); 130.15 (C-6"); 118.81 (C-5"); 118.72 ( C-2); 118.44 (C-3"); 117.67 (C-3'); 111.07 (C-4'); 57.87 (C-4, CH₂OH).
**IR (aufgenommen als Film):** 3329 (OH), 3122 (=CH ), 2875 (CH₂), 1638 (C=O), 1566 (C=C) cm⁻¹.

### 1.3 Synthese von 1-(2-Hydroxyphenyl)-3-(4-methoxyphenyl)propenon (CH-1)

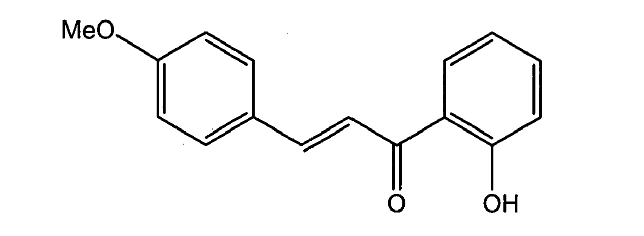

Zu einer Mischung, bestehend aus 4-Methoxybenzaldehyd und 2-Hydroxyacetophenon im molaren Verhältniss 1:1 in 40 ml Ethanol wird Bariumhydroxyd hinzugefügt und entsprechend der Arbeitsvorschrift AAV2 umgesetzt.
Summenformel: C₁₆H₁₄O₃; Molmasse: 254
Gemessene Werte für
Fp: 89-91°C.

Ausbeute: 43 %.

| Elementaranalyse: | | |
|---|---|---|
| Berechnet für die Summenformel C₁₆H₁₄O₃: | 75,59 % C ; | 5,51 % H |
| Gefunden: | 75,54 % C ; | 5,47 % H |

Charakterisierende spektrometische Daten:
**¹H-NMR (CDCl₃):** 12.90 (s, 1H, OH); 7.93 (d, 1H, H-3); 7.60 (m, 1H, H-6" ); 7.56 (d, 1 H, H-2); 7.40 (m,1 H, H-4"); 7.30 (d,2H, H-6' / H-2'); 7.01 (m, 1 H, H-5"); 6.92 (m, 1 H, H-3" ); 6.72 (m, 2H, H-3'/ H-5'); 3.92 (s, 3H, CH₃O).
**¹³C-NMR (CDCl₃):** 193.68 (CO); 163.57 (C-4'); 162.05 (C-2"); 145.38 (C-3); 136.17 (C-4"); 130.59 (C-6"); 129.56 (C-2'/C-6'); 127.33 (C-1'); 120.13 (C-1"); 118.78 ( C-2); 118.59 (C-5"); 117.57 (C-3"); 114.53 (C-3'/ C-5'); 55.46 (C-4, CH₃O).
**NMR-DEPT (CDCl₃):** 145.39 (C-3); 136.17 (C-4" ); 130.59 (C-6"); 129.56 (C-2'/C-6'); 118.79 ( C-2); 118.59 (C-5"); 117.56 (C-3"); 114.53 (C-3'/ C-5'); 55.47 (C-4, CH₃O).
**IR (aufgenommen als Film):** 3330 (OH), 3120 (=CH ), 2975 (CH₃), 1640 (C=O), 1564 (C=C) cm⁻¹.

### 1.4 Synthese von 3-(4-Hydroxy-3-methoxyphenyl)-1-(2-hydroxyphenyl)-propenon (Q-20).

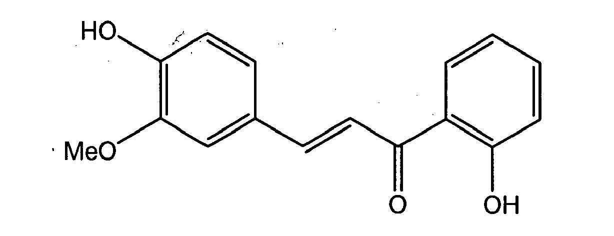

0,01mol (1,52g) Vanillin und 0,01mol (1,36g, d.h. 1,2 ml) o-Hydroxyacetophenon werden in 10 ml absolutem Ethanol gelöst. Anschließend werden 2 ml wasserfreies Piperidin hinzugefügt und entsprechend der Arbeitsvorschrift AAV3 umgesetzt. Zur chromatographischen Aufarbeitung wird als Eluent eine Mischung aus Toluol und Ethylacetat im Verhältnis 9:1 verwendet.
Summenformel: C₁₆H₁₄O₄; Molmasse: 270
Gemessene Werte für die
Ausbeute: 65 %

| Elementaranalyse: | | |
|---|---|---|
| Berechnet fürdie Summenformel C₁₆H₁₄O₄: | 71,11 % C ; | 5,19 % H |
| Gefunden: | 71,09 % C ; | 5,15 % H |

Charakterisierende spektrometrische Werte:
**¹H-NMR (CDCl₃):** 13.00 (s, 1 H, OH); 7.93 (d, 1 H, H-3); 7.60 (m, 1 H, H-6" ); 7.51 (d, 1 H, H-2); 7.32 (m, 1 H, H-4" ); 7.22 (m, 1 H, H-5" ); 7.20 (m, 1 H, H-3" ); 7.17 (m, 1H, H-6'); 7.10 (m, 1H, H-2'); 7.00 (m, 1H, H-5'); 6.1 (s, 1H, OH); 3.94 (s, 3H, CH₃O).
¹³C-NMR (CDCl₃): 193.62 (CO); 163.53 (C-2"); 148.73 (C-3'); 148.51 (C-4'); 146.87 (C-1'); 145.89 (C-3); 136.20 (C-4"); 129.56 (C-6"); 123.79 (C-2); 118.79 (C-5"); 118.60 (C-6'); 117.46 (C-5'); 114.99 (C-3"); 110.27 (C-2'); 56.06 (MeO).
**NMR-DEPT (CDCl₃):** 145.90 (C-3); 136.21 (C-4"); 129.56 (C-6"); 123.80 (C-2); 118.80 (C-5"); 118.60 (C-6'); 117.45 (C-5'); 114.99 (C-3"); 110.26 (C-2'); 56.06 (CH₃O).
**IR (Nujol):** 3339 (OH), 1637 (C=O), 1552 (C=C) cm⁻¹.

### 1.5 Synthese von 1-(2-Hydroxyphenyl)-3-[3-methoxy-4-(3-methylbut-2-enyloxy)phenyl]propenon (Q-21).

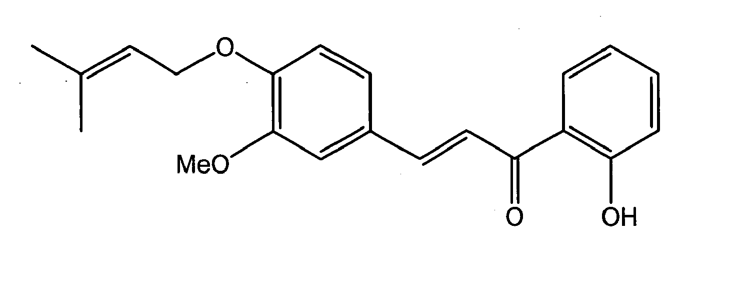

0,005 mol (1,1g) 3-Methoxy-4-(3-methylbut-2-enyloxy)benzaldehyd und 0,005 mol (0,68g, d.h. 0,6 ml) o-Hydroxyacetophenon werden in 10 ml absolutem Methanol gelöst. Anschließend werden 2 ml wasserfreies Piperidin hinzugefügt und entsprechend der Arbeitsvorschrift AAV3 umgesetzt.
Summenformel: C₂₁H₂₂O₄; Molmasse: 338
Gemessene Werte für die
Ausbeute: 85 %

| Elementaranalyse: | | |
|---|---|---|
| Berechnet für die Summenformel C₂₁H₂₂O₄: | 74,56 % C ; | 6,51 % H |
| Gefunden: | 74,54 % C ; | 6,48 % H |

Charakterisierende spektrometische Werte:
**¹H-NMR (CDCl₃):** 12.90 (s, 1H, OH); 7.95 (d, 1H, H-3); 7.64 (m, 1H, H-6" ); 7.56 (d, 1 H, H-2); 7.37 (m, 1 H, H-4" ); 7.20 (m, 1 H, H-5" ); 7.10 (m, 1 H, H-3" ); 6.98 (m, 1 H, H-6'); 6.95 (m, 1 H, H-2'); 6.92 (m, 1 H, H-5'); 5.5 (m, 1 H, H-5); 4.61 (d, 2H, H-4); 1.77 (s, 3H, CH₃-prenyl); 1.71 (s, 3H, CH₃-prenyl).
¹³C-NMR (CDCl₃): 193.61 (CO); 163.57 (C-2"); 154.01 (C-3'); 150.33 (C-4'); 148.41 (C-3); 145.82 (C-4"); 138.55 (C-1"); 136.17 (C-6" ); 130.73 (C-6); 129.55 (C-2 ); 127.43 (C-1'); 123.55 (C-5); 120.13 (C-5" ); 119.28 (C-6'); 118.75 (C-3" ); 112.56 (C-5'); 110.45 (C-2'); 65.83 (C-4); 56.04 (MeO); 25.88 (CH₃-prenyl); 18.32 (CH₃-prenyl).

**NMR-DEPT (CDCl₃):** 148.39 (C-3); 145.82 (C-4"); 136.17 (C-6"); 129.55 (C-2); 123.55 (C-5); 120.01 (C-5"); 118.75 (C-6'); 117.61 (C-3"); 112.56 (C-5'); 110.44 (C-2'); 65.83 (C-4); 56.04 (CH₃O); 25.88 (CH₃-prenyl); 18.33 (CH₃-prenyl).
**IR (Nujol):** 3300 (OH), 1635 (C=O), 1550 (C=C) cm⁻¹.

### 1.6 Synthese von 3-[3-Methoxy-4-(3-methylbut-2-enyloxy)phenyl]-1-[2-(3-methylbut-2-enyloxy)phenyl]propenon (LH2)

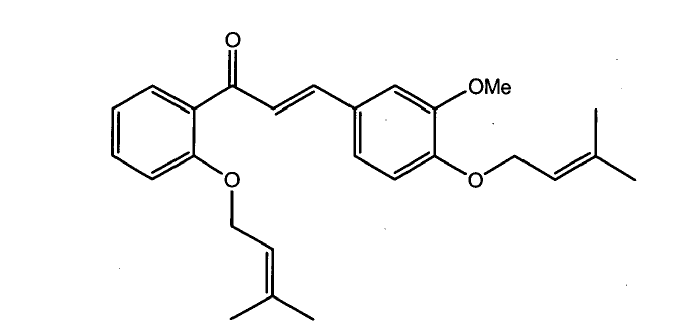

0,01mol 3-Methoxy-4-(3-methylbut-2-enyloxy)benzaldehyd und 0,01mol 1-[2-(3-Methylbut-2-enyloxy)phenyl]ethanon werden entsprechend der Arbeitsvorschrift AAV3 umgesetzt.
Summenformel: C₂₅H₃₀O₄ ; Molmasse: 394
Ausbeute: 75 %
Charakterisierende spektrometrische Werte:
**¹H-NMR (CDCl₃):** 7.52 (d, 1 H, H-3); 7.46 (m, 1 H, H-6" ); 6.80 (d, 1 H, H-2); 7.38 (m, 1 H, H-4" ); 7.37 (m, 1 H, H-5" ); 7.35 (m, 1 H, H-3" ); 6.93 (m, 1 H, H-6'); 6.91 (m, 1 H, H-2'); 6.88 (m, 1 H, H-5'); 5.44 (m, 1 H, H-8); 5.43 (m, 1 H, H-5); 4.61 (d, 2H, H-7); 4.54 (d, 2H, H-4); 3.88 (s, 3H, MeO); 1.72 (s, 6H, CH₃-prenyl); 1.69 (s, 6H, CH₃-prenyl).

### 1.7 Synthese von 2-[3-Methoxy-4-(3-methylbut-2-enyloxy)phenyl]chroman-4-on (Q-25).

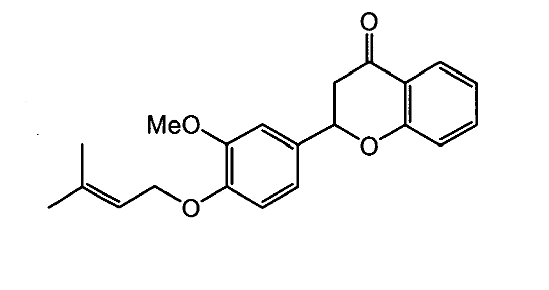

8,5 mmol (2,9g) 1-(2-Hydroxyphenyl)-3-[3-methoxy-4-(3-methylbut-2-enyloxy)-phenyl]propenon und 153 mmol (20,8g) Natriumacetat 3H₂O werden in 50ml Ethanol gelöst und die Reaktionsmischung 8 Stunden unter Rückfluß erhitzt. Anschließend wird die Reaktionsmischung auf Eiswasser gegossen und mit Dichlormethan (3 x 15ml) extrahiert. Die vereinigten organischen Phasen dieser Extraktion werden mit Wasser gewaschen, über Natriumsulfat getrocknet, im Vakuum eingeengt und säulenchromatographisch gereinigt (n-Heptan-Ethylacetat-Gemisch im Verhältnis 8:2).
Summenformel: C₂₁H₂₂O₄; Molmasse: 338
Ausbeute: 83 %

| Elementaranalyse: | | |
|---|---|---|
| Berechnet für die Summenformel C₂₁H₂₂O4: | 74,56 % C ; | 6,51 % H |
| Gefunden: | 74,51 % C ; | 6,47 % H |

Charakterisierende spektrometische Werte:
**¹H-NMR (CDCl₃):** 1.73 (s, 3H, C-1, CH₃-prenyl); 1.77 (s, 3H, C-1', CH₃-prenyl); 5.51 (m, 1H, H-3); 4:60 (d, 2H, H-4, ³J₃₋₄ 6.6 Hz); 7.01 (d, 1 H, H-7, ⁴J₇₋₉ 2.2 Hz ); 6.97 (dd, 1H, H-9, ³J_{9,-10} 8.2 e ⁴J₇₋₉ 2.2 Hz); 6.90 (d, 1 H, H-10, ³J₉₋₁₀ 8.2 Hz ); 5.41 (dd, 1H, H-11, ³J_{11-12 ax.}13.4; ³J_{11-12 eq.} 3.0 Hz ); 3.11 (dd, 1H, H-12, CH₂, axial ²J_{A-B} 16.8; 13.4 Hz); 2.87 (dd, 1H, H-12, CH₂ eq. ²J_{A-B} 16.8; 3.0 Hz); 7.92 (dd, 1 H, H-15, ³J₁₅₋₁₆ 8.2; ⁴J₁₅₋₁₇ 8.0 Hz); 7.50 (ddd, 1H, H-17, ³J 7.2, ³J 8.2, ⁴J₁₅₋₁₇ 2.0 Hz); 7.04 (m, 2H, H-16 e H-18); 3.90 (s, 3H, MeO).
**¹³C-NMR** (500 MHz) **(CDCl₃):** 18.3 (C-1, CH₃-prenyl); 25.9 (C-1', CH₃-prenyl); 137.8 (C-2); 119.8 (C-3); 65.8 (C-4); 148.8 (C-5); 149.7 (C-6); 109.7 (C-7); 131.1 (C-8); 118.7 (C-9); 112.8 (C-10); 79.7 (C-11); 44.6 (C-12); 192.1 (CO); 121.0 (C-14); 127.1 (C-15); 121.6 (C-16); 136.2 (C-17); 118.2 (C-18); 161.6 (C-19); 56.0 (MeO).
**NMR-DEPT (CDCl₃):** 136.20 (C-17); 127.06 (C-15); 121.59 (C-16); 119.75 (C-3); 118.72 (C-9); 118.16 (C-18); 112.83 (C-10); 109.60 (C-7); 79.8 (C-11); 65.80 (C-4); 55.99 (MeO); 44.61 (C-12); 25.87 (C-1'); 18.29 (C-1).
**IR (Nujol):** 1690 (C=O), 1550 (C=C) cm-1.

### 1.8 Synthese von 2-{1-[2-(3-Methylbut-2-enyloxy}phenyl]ethyliden}-malononitril (L5)

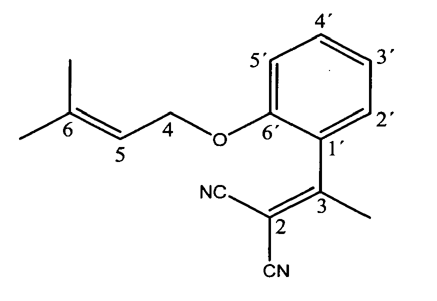

0,01mol (1,84g) 1-[2-(3-Methylbut-2-enyloxy)phenyl]ethanon und 0,01mol (0,66g) Malononitril werden entsprechend der Arbeitsvorschrift AAV4 umgesetzt.
Der ölige Rückstand wird chromatographisch aufgearbeitet. Bei der chromatographischen Aufarbeitung wird als Eluent eine Mischung aus Toluol und Ethylacetat im Verhältnis von 9:1 verwendet.
Summenformel: C₁₆H₁₆N₂O ; Molmasse: 252
Ausbeute: 80 %

| Elementaranalyse: | | | |
|---|---|---|---|
| Berechnet für die Summenformel C₁₆H₁₆N₂O: | 76,17 % C ; | 6,39 % H ; | 11,09 % N |
| Gefunden: | 76,19 % C ; | 6,41 % H ; | 10,98 % N |

Charakterisierende spektrometrische Werte:
**¹H-RMN (DMSO-d₆):** 7,2 (d, 2H, H-3'/H-5'), 6,8 (d, 2H, H-2'/H-4'), 5,2 (m, 1H, H-5), 4,4 (d, 2H, H-4), 2,4 (s, 3H, CH₃(C=C(CN)₂)), 1,8 (d, 6H, CH₃, CH₃).

### 1.9 Synthese von 2-[1-(4-Hydroxyphenyl)ethyliden]malonitril (OK)

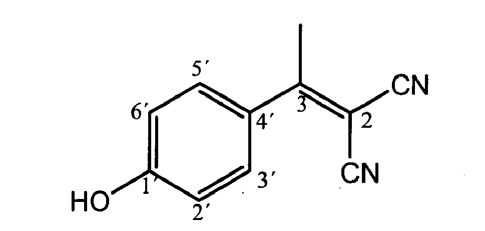

0,01mol (1,36g) 1-(4-Hydroxyphenyl)ethanon und 0,01mol (0,66g) Malononitril werden entsprechend der Arbeitsvorschrift AAV4 umgesetzt.
Die organische Phase wird anschließend mit einer NaCI-Lösung gewaschen und darauffolgend mit Natriumsulfat getrocknet. Nach Filtration wird die Lösung unter vermindertem Druck eingeengt. Dabei fällt ein gelbes Produkt aus, das aus Ethanol umkritalisiert wird.
Summenformel: C₁₁H₈N₂O; Molmasse: 184,17
Ausbeute: 40%
Fp: 97°C

| Elementaranalyse: | | | |
|---|---|---|---|
| Berechnet für die Summenformel C₁₁H₈N₂O: | 71,73 % C ; | 4,38 % H ; | 15,20 % N |
| Gefunden: | 71,78 % C ; | 4,39 % H ; | 15,17 % N |

Charakterisierende spektrometrische Werte:
**¹³C-NMR (CDCl₃) :** 174,8 (C-3), 160,0 (C-1'), 129,8 (C-3'/5'), 127,46 (C-4'), 115,9 (C-2'/6'), 113,6 (CN), 113,1 (CN), 81,2 (C-2), 23,9 (CH₃).
**IR (film):** 3496 (OH), 3028 (=C-H), 2962 (CH₃), 2229 (CN) cm⁻¹.
**MS (70eV):** 184 M+ , 167 (M+ - 17), 156 (M+ - 28).

### 1.10. Synthese von 2-{1-[4-(3-Methylbut-2-enyloxy)phenyl]ethyiliden}-malononitril (LPK1)

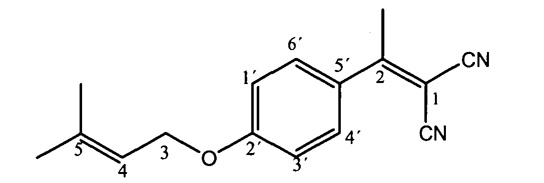

0,01mol (2,04g) 1-[4-(3-Methylbut-2-enyloxy)phenyl]ethanon und 0,01mol (0,66g) Malononitril werden entsprechend der Arbeitsvorschrift AAV4 umgesetzt.
Die organische Phase wird anschließend mit einer NaCl-Lösung gewaschen und darauffolgend mit Natriumsulfat getrocknet. Nach Filtration wird die Lösung unter vermindertem Druck eingeengt. Dabei fällt ein braunes Produkt aus, das aus Ethanol umkritalisiert wird.
Summenformel: C₁₆H₁₆N₂O ; Molmasse: 252,29
Ausbeute: 70%
Fp: 71 °C

| Elementaranalyse: | | | |
|---|---|---|---|
| Berechnet für die Summenformel C₁₆H₁₆N₂O: | 76,17 % C ; | 6,39 % H ; | 11,09 % N |
| Gefunden: | 76,15 % C ; | 6,35 % H ; | 11,04 % N |

Charakterisierende spektrometrische Werte:
**¹H-NMR (DMSO-d₆):** 7,6 (d, 2H, H-2'/H-4'), 7,0 (d, 2H, H-1'/H-5'), 5,5 (m, 1H, H-4), 4,6 (d, 2H, H-3), 2,6 (s, 3H, CH₃(C=C(CN)₂)), 1,8 (s, 3H, CH₃), 1,7 (s, 3H, CH₃).
**¹³C-NMR (CDCl₃) :** 173,8 (C-2), 162,7 (C-2'), 139, 2 (C-1), 129,7 (C-4'/6'/5'), 118,8(C-4), 115,0 (C-1'/C-3'), 114,8 (CN), 114,2 (CN), 81,6 (C-1), 65,1 (C-3), 25,7 (CH₃(C=C(CN)₂), 23,77 (CH₃), 23,72 (CH₃).
**IR (film):**3066, 3026 (=C-H), 2976 (CH₃), 2877 (CH₂), 2222 (CN), 1248 (C-O) cm⁻¹.
**MS (70eV):** 252 M+, 226 (M+-26), 69 (Prenylgruppe).

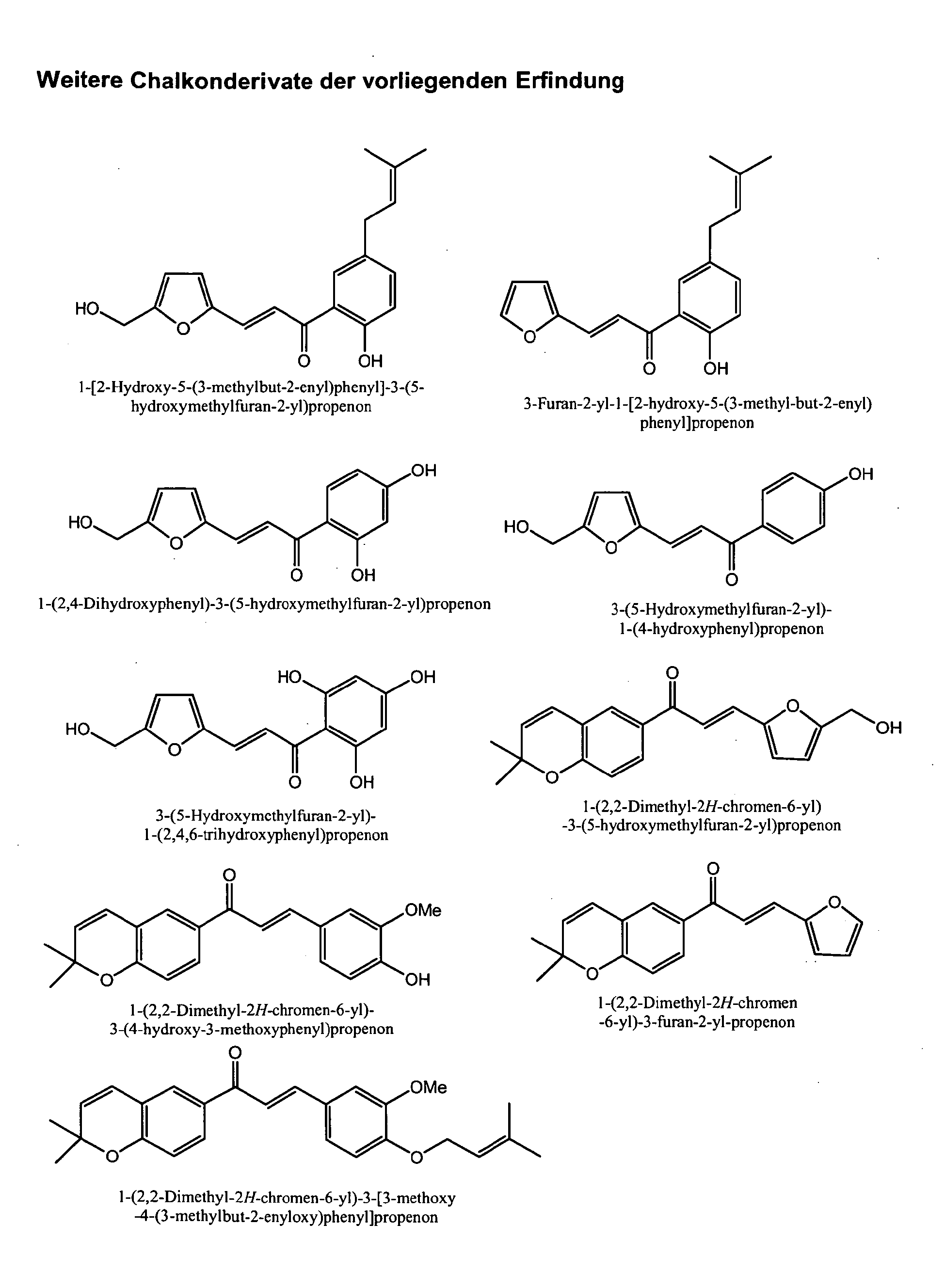

### Nachweis der biologischen/pharmakologischen Wirkungen der erfindungsgemässen Verbindungen

### 1. Antitumorale Wirkung der erfindungsgemässen Verbindungen

### Testverfahren: Hemmwirkung auf humane Zell-Linien

Die folgenden erfindungsgemässen Verbindungen wurden in den Versuchen untersucht:HMF-2, HMF-3, Q20 und Q25.
Die Verbindungen wurden in Dimethylsulfoxid (DMSO) als konzentrierte Stammlösungen (100-400fach) gelöst und für Proliferationstests in Zellkulturmedium verdünnt.

### Verwendete Zelllinien:

Mit Ausnahme von SIM, KAL Ewingsarkom (in Wien etabliert) und LAN Neuroblastom (University of Los Angeles) wurden die Zell-Linien von der American Type Culture Collection (ATCC, Rockville, MD) bezogen und zum Erhalt von konfluenten Monolayerkulturen in RPMI-1640 Bikarbonatmedium (Seromed, Berlin, Deutschland) in einem Zellkulturschrank (5% CO₂, 37°C) kultiviert. Mit der Ausnahme der murinen NlH3T3 Fibroblasten sind alle verwendeten Zell-Linien humanen Ursprungs. Die Zellen wurden auf Verunreinigung durch Mycoplasmen untersucht. Das Medium wurde mit 10% hitzeinaktiviertem foetalem Kälberserum (Seromed) and 4 mM Glutamin ergänzt. Die Zellen wurden durch Trypsinierung subkultiviert (0.03% Trypsin enthaltend 0.02% EDTA, dreimal pro Woche). Die Trypsinierung wurde durch Verwendung vom komplettem Zellkulturmedium gestoppt. Die Zellzahl wurde unter Verwendung eines TOA Sysmex Microzellzählers (TOA, Tokyo, Japan) bestimmt.

### Chemikalien und Lösungen:

Falls nicht anders angegeben, wurden alle Chemikalien von Sigma (St. Louis, MO) bezogen. Der modifizierte MTT Test EZ4U wurde von Biomedica, Wien, Österreich bezogen.

### Zehllzahlbestimmung in einem Proliferationstest:

Die Zellen wurden in Makroplatten mit 6 Vertiefungen (Greiner, Kremsmünster, Österreich) in einer anfänglichen Zelldichte von 200 000 Zellen/Vertiefung in 4 ml Medium unter Zellkulturbedingungen in Anwesenheit oder Abwesenheit der Testverbindungen (doppelte Ansätze) kultiviert. Nach Inkubation über die jeweilige Zeitdauer wurden die Zellen mit Medium gespült um abgelöste tote Zellen zu entfernen und die intakten Zellen wurden durch Trypsinierug geerntet. Nach Zentrifugation wurden die Zellen in PBS resuspendiert und die Zellzahl in einem Microzellzähler (TOA, Tokio, Jpn) in dreifachen Ansätzen bestimmt. Die Ergebnisse sind als Prozentsatz der Zellen in Anwesenheit der Testverbindungen in Bezug auf Mediumkontrollen (100%) angegeben.

### Chemosensitivitätstests:

10⁴ Zellen/well wurden in Mikrotiterplatten mit 96 Vertiefungen (100 µl Medium) verteilt und die zu testenden Verbindungen wurden in einem weiteren Volumen von 100 µl hinzugegeben. Alle Verbindungen wurden entweder in 3 fixen Konzentrationen eingesetzt (für Tabellen 1-3) oder in 10 Stufen verdünnt unter Verwendung von Zweifachverdünnungsschritten zur Bestimmung der IC50-Werte (dreifache Ansätze, in den meisten Fällen vierfache Ansätze). Die Platten wurden, mit der Ausnahme der Tests zur Untersuchung des Zusammenhangs von Applikationsdauer und Zellreaktion, 4 Tage lang unter Zellkulturbedingungen inkubiert und die Zellviabilität wurde unter Verwendung eines modifizierten MTT Tests bestimmt (Formazan-Farbstoffbildung aus Tetrazoliumsalzen wie MTT, XTT, WST-1 und anderen durch das mitochondriale Reduktionsystem Succinattetrazolium Reductase EC 1.3.99.1.; EZ4U nichtradioaktiver Zellproliferations- und Cytotoxizitätstest, Biomedica, Wien, Österreich; Testmanual unter: www.bmgrp.com). Hierbei wurde die mitochondriale Aktivität und dadurch die Zellviabilität/Zellzahl bestimmt.
Kurz gesagt wurde das gelieferte Substrat in einer Aktivatorlösung gelöst und 20 µl der Mischung wurden für 2 Stunden pro Vertiefung hinzugegeben (Gesamtvolumen 200 µl). Die optische Dichte wurde bei 450 nm unter Verwendung einer leeren Vertiefung als Referenz in einem Mikroplattenlesegerät (Eurogenetics, Brüssel, Belgien) bestimmt. Für jede Zell-Line wurden Vertiefungen verwendet um das MTT-Signal der Mediumkontrollen ohne Testsubstanzen zu bestimmen. Die Proliferation in den Test-Vertiefungen wurde in Bezug auf diese Kontrollwerte (100%) errechnet. Die Testergebnisse wurden zwischen 0,3 und 1,5 optischer Dichte für langsam bzw. schnellproliferierende Zellen erfasst.
Diese Dosis-Wirkungs-Beziehungen wurden verwendet um die IC₅₀ (Konzentration bei der das Wachstum um 50% gehemmt wird) unter Verwendung von PRISM Scientific Software (GraphPad Inc., San Diego, CA) zu berechnen.

### Testergebnisse:

Tabellen 1 und 2 und Tabellen 4 und 5 beruhen auf der Methode, die unter Chemosensitivitätstest beschrieben is; die Tabelle 3 beruht auf der Methode, die unter Zellzahlbestimmung beschrieben ist.
Die Ergebnisse antitumoraler Tests sind in den Tab. 1. bis Tab.3 aufgelistet.

**Tabelle 1:**

| Krebszell-Linien: Colo205 (Colon), T47D (Brust), MIAPaCa2 (Pankreas) Verbindung HMF-2. Zellwachstum in % der Mediumkontrolle. | | | | |
|---|---|---|---|---|
| Krebstyp | Zell-Linien | Konzentration 1 (10 µg/ml) | Konzentration 2 (5 µg/ml) | Konzentration 3 (2,5 µg/ml) |
| Colon | Colo205 | 41,30% | 78,6% | 84,80% |
| Mamakarzinom | T47D | 4,90% | 25,60% | 28,10% |
| Pankreas | MIAPaC a2 | 74,60% | 91,10% | 84,30% |
| Fibroblasten | WI38 | 10,60% | 92,90% | 104,7% |

Die Verbindung HMF-2 zeigt cytocide Aktivität gegen alle getesteten Zell-Linien. Besonders stark selektiv wirkt die Substanz HMF-2 gegen Brust-Krebszellen (T47D) bei einer Konzentration zwischen 2,5 und 5 µg/ml, ohne dabei die Fibroblasten zu schädigen.

**Tabelle 2:**

| Krebszell-Linien: Colo205 (Colon), T47D (Brust), MlAPaCa2 (Pankreas) Verbindung HMF-3 Zellwachstum in % der Mediumkontrolle. | | | | |
|---|---|---|---|---|
| Krebstyp | Zell-Linien | Konzentration 1 (10 µg/ml) | Konzentration 2 (5 µg/ml) | Konzentration 3 (2,5 µg/ml) |
| Colon | Colo205 | 82,60% | 68% | 85,90% |
| Mamakarzinom | T47D | 50,20% | 50,00% | 39,10% |
| Pankreas | MIAPaC a2 | 50,60% | 81,80% | 84,10% |
| Fibroblasten | Wl38 | 81,50% | 64,70% | 92% |

Die Verbindung HMF-3 zeigt cytocide Aktivität gegen alle getesteten Zell-Linien. Sie wirkt selektiv gegen Breast-Krebszellen (T47D) bei einer Konzentration zwischen 2,5 µg/m, ohne dabei die Fibroblasten zu schädigen.

**Tabelle 3:**

| Antiproliferativen Aktivität der Verbindungen HMF-2 und HMF-3. Inhibition des Zellenwachstums (in %) der Krebszell-Linien Mama (Brust T47D) und Colon (Colo205) bei einer Konzentration von 50 µg/ml | | | |
|---|---|---|---|
| Verbindungen | BT20(Brust) | T47D(Brust) | Colo205(Colon) |
| HMF-2 | 74% | 75,60% | 35,6% |
| HMF-3 | 15,70% | 57,00% | 3,50% |

Die Verbindung HMF-2 zeigte eine erhebliche antiproliferative Aktivität gegen die Krebszellen-Linien BT20 Brust und T47D Brust, während die Verbindung HMF-3 eine mittlere antitumorale Aktivität gegen die Brust-Zellen (T47D) besitzt.

**Tabelle 4:**

| Vergleich der IC₅₀-Werte (µg/ml) der Verbindungen HMF-2 und HMF-3 gegen eine Reihe von Krebszell-Linien und Fibroblasten. | | | |
|---|---|---|---|
| Zell-Linien | Herkunft | HMF-2 | HMF-3 |
| U87MG | Astrocytom | >200 | >200 |
| CRO2B | Carcinoid | 50 | > 200 |
| MIAPaCa2 | Pancreas | 10 | > 200 |
| SIM | Sarcom | >200 | >200 |
| KAL | Sarcom | 45 | >200 |
| EW-7 | Sarcom | 0.3 | 5.25 |
| SKOV3 | Ovar | 36 | >200 |
| SW620 | Colon | 0.85 | 9.7 |
| CaCo-2 | Colon | 17.5 | >200 |
| J82 | Blase | 47 | >200 |
| LAN1 | Neuroblastom | 4.25 | 20.0 |
| LNCaP | Prostata | 3.25 | 9.5 |
| NlH3T3 | Fibroblasten | 1.25 | 11.0 |
| WI38 | Fibroblasten | 4.9 | 20.0 |

Die Verbindung HMF-2 wirkt stark aktiv gegen 10 Humankrebszell-Linien; besonders stark wirksam gegen die Zell-Linien MIPaCa2, EW-7, SW620, CaCo-2, LAN1 und LNCaP. Die Verbindung HMF-2 wirkt stark aktiv gegen 4 Humankrebszellen-Linien; besonders stark wirksam gegen die Zell-Linien EW-7, SW620, LAN1 und LNCaP. Beide Verbindungen zeigten auch starke Aktivität gegen die hier untersuchten Fibroblasten, wobei die Verbindung HMF-3 eine niedrigere (höhere IC-50 Werte) aufweist, d.h. sie ist etwas selektiver als HMF-2.

**Tabelle 5:**

| Vergleich der IC₅₀-Werte (µg/ml) der Verbindungen Q25 und Q20 gegen eine Reihe von Krebszell-Linien und Fibroblasten | | | |
|---|---|---|---|
| Zell-Linien | Herkunft | Q25 | Q20 |
| Colo205 | Colon | 75 | 27 |
| SW620 | Colon | 84 | 17 |
| HT29 | Colon | 18 | 16 |
| CaCo-2 | Colon | 18 | 12 |
| MDA-MB435 | Mamma | 34 | 24 |
| BT20 | Mamma | 45 | 33 |
| T47D | Mamma | 10 | 25 |
| DU 145 | Prostata | 15 | 16 |
| CR02B | Carcinoid | 6 | 12 |
| BxPC3 | Pankreas | 80 | 33 |
| MiaPaCa-2 | Pankreas | 57 | 18 |
| WI-38 | Fibroblasten | 8.2 | 8.3 |

Die Verbindung Q20 wirkt stark aktiv gegen 11 Humankrebszell-Linien; besonders stark wirksam gegen die Zell-Linien MlPaCa2, SW620, HT29, CaCo-2, MDA-MB435, CRO2B, DU145, T47D und MiaPaCa-2. Die Verbindung Q25 wirkte sehr aktiv gegen 7 Humankrebszellen-Linien; besonders stark wirksam gegen die Zell-Linien HT29, CaCo-2, MDA-MB435, DU145, T47D und CRO2B. Beide Verbindungen zeigen starke Aktivität gegen Fibroblasten.

### 2. Radikalfänger-Wirkung der erfindungsgemässen Verbindungen

### Testverfahren: Screening-Untersuchung mit DPPH-Radikalen

Getestet wurden die 6 Verbindungen HMF2, HMF-3, CH-1, L5, Q20 und Q25.
Die Radikalfängereigenschaften wurden durch den DPPH-Test ermittelt, wobei sich die UV/VIS-Absorption des freien stabilen Radikals DPPH (Diphenyl-β-picrylhydrazin) in Abhängigkeit vom Zustand von Radikalfängern ändert. Die Auswertung erfolgt qualitativ (dünnschichtchromatographisch) und quantitativ (photometrisch) mit Ascorbinsäure als Vergleichssubstanz (siehe Molyneux P. (2004) The use of the stabel free radical diphenylpicrylhydrazyl (DPPH) for estimating antioxidant activity. Songklanakarin J Sci Technol. 26(2): 211-219).

### Testergebnisse:

Die Verbindung Q20 zeigt in den Konzentrationen 500 und 1000µg/ml einen sehr hohen Radikalfänger-Effekt von 98%, die Verbindung L5 in den Konzentrationen 500 und 1000µg/ml einen etwas geringeren Radikalfänger-Effekt von 60%

### 3. Antimikrobielle Wirkung der erfindungsgemässen Verbindungen

### Testverfahren: Bestimmung der minimalen Hemmkonzentration (MHK)

Das Inokulum für die Tests wurde durch Verdünnung der Mikroorganismen in wässriger 0,85% NaCI-Lösung hergestellt, die Verdünnung auf die Stufe 0,5 nach McFarland eingestellt und die Konzentration spektrometrisch bei einer Wellenlänge von 580nm überprüft. Die Zellsuspensionen wurden zur Verwendung in den Tests auf 10⁴ UFC/ml verdünnt. Die MHK-Tests erfolgten (Vorschrift nach J. N. P. Eloff, Planta Medica, Vol. 64, 1998, p 711-713) in Mikrotiterplatten mit 96 Vertiefungen. Serienverdünnungen der Konzentrationen lagen im Bereich 1,00 - 0,0015 mg/mL. Als Kontrollsubstanzen dienten Chloramphenicol bzw. Nistatin (Merck) in einer Konzentration von 0,062 - 0,005 mg/ml bzw. 0,0125 - 0,001 mg/ml. Das Inokulum (100 µl) wurde in alle Vertiefungen gegeben und die Platten für 48h bei 36°C inkubiert. Die antimikrobielle Aktivität wurde durch Zugabe von 20 µl einer 0,5% igen wässrigen Lösung von TTC (Tetrazoliumchlorid, Merck) pro Vertiefung detektiert. Die minimale Hemmkonzentration (MHK) wird als die niedrigste Konzentration der Verbindung definiert die das sichtbare Wachstum hemmt (durch TTC-Färbung indiziert).

### Testergebnisse:

Die Ergebnisse der antimikrobiellen Tests werden in Tab. 6 und Tab. 7 gezeigt.

**Tabelle 6: MHK-Werte (in mg/ml) der Verbindungen Q21 und Q20:**

| Microorganismen | Q21 | Q20 | Vergleichsubstanz Chloramphenicol |
|---|---|---|---|
| *Bacillus subitilis* | >1,0 | 0,5 | 0,02 |
| *Micrococcus luteus* | >1,0 | 1,0 | 0,05 |
| *Rhodococcus equi* | >1,0 | >1,0 | 0,04 |
| *S. falcium* | 0,8 | 0,3 | 0,12 |
| *Salmonella sp* | 0,9 | >1,0 | 0,06 |
| *Escherichia coli* | >1,0 | >1,0 | 0,04 |
| *S. aureus* | >1,0 | 1,0 | 0,02 |
| *E. hirae* | >1,0 | >1,0 | - |
| *S. epidermides* | >1,0 | 0,5 | 0,04 |
| *Candida albicans* | 0,6 | 0,3 | 0,05** |

| | | | |
|---|---|---|---|
| ** Vergleichsubstanz gegen *Candida albicans* Nistatin. | | | |

**Tabelle 7: MHK-Werte (in mg/ml) der Verbindungen OK und LPK1**

| Microorganismen | OK | LPK1 | Vergleichsubstanz Chloramphenicol |
|---|---|---|---|
| *S. epidermides* | 0,03-0,06 | 0,5-1,0 | 0,04 |
| *E. coli* | 0,5-1,0 | > 1,5 | 0,04 |
| *Rhodococcus equi* | 0,5-1,0 | > 1,5 | 0,04 |
| *Candida albicans* | 0,5-1,0 | > 1,5 | 0,12** |
| *B. subtilis* | 0,12-0,25 | > 1,5 | 0,02 |
| *Micrococcus luteus* | 0,12-0,25 | > 1,5 | 0,05 |
| *E. faecium* | 0,12-0,25 | > 1,5 | 0,12 |
| *Salmonella* | 0,25-0,5 | > 1,5 | 0,06 |
| *S. aureus* | 0,25-0,5 | > 1,5 | 0,02 |
| *S*.falcium | 0,5-1,0 | > 1,5 | 0,12 |

| | | | |
|---|---|---|---|
| ** Vergleichsubstanz gegen *Candida albicans* Nistatin. | | | |

### 4. Antiseptische Wirkung der erfindungsgemässen Verbindungen

### Testverfahren: Mäuseohrtest (in vivo)

Mäuseohren wurden in Dreifachbestimmungen mit *Staphylococcus aureus* (Stamm ATCC 6538) infiziert. Die Zugabe der Testverbindungen (50µl einer 10%igen wässrigen Lösung) erfolgte nach 1,5 Stunden. Die Lösung unter Ultraschall (15 min) mit 5% DMSO und der Ausstrich auf Agar-Platten 3 h danach; die Ermittlung der Keimzahl im Vergleich zum infizierten unbehandelten Versuchstier nach 18 und 24 Stunden.

### Testergebnisse:

- HMF2: totale Verhinderung des Keimwachstums (+++)
- L5: totale Verhinderung des Keimwachstums (+++)
- CH-1: Keimwachstum deutlich reduziert aber nicht völlig unterdrückt (++)
- Q20: Keimwachstum auf 30 % reduziert (++)
- Q25: Keimwachstum auf 30 % reduziert (++)

Die vorstehenden Testergebnissen zeigen die hervorragende antimikrobielle (gegen Bakterien und Pilze) antiproliferative Wirkung, Radikalfängerwirkung und antiseptische Wirkung der erfindungsgemäßen Verbindungen.

Die erfindungsgemässen Verbindungen können somit zur Therapie und Prophylaxe von malignen Tumoren im Menschen wie z.B. soliden Tumoren wie Coloncarzinoma, Rektumkarzinoma, Magenkrebs, Krebs der Schilddrüse, Zungenkrebs, Blasenkrebs, Chorionkarzinom, Leberkrebs, Gebärmutterkrebs, Prostatakarzinom, Pharyngealkarzinom, Lungenkrebs, Mammakarzinom, malignes Melanom, Karposi Sarkom, Hirntumore, Neuroblastom, Ovarialkarzinom, Hodenkrebs, Osteosarkom, Pankreaskrebs, Nierenkrebs,Hypernephroma, und Angioendothelioma; und hematopoietische maligne Tumore wie Leukämie oder Lymphoma, insbesondere bei Lungenkrebs, Mammakarzinom, Melanoma, Leukämie, Colonkarzinom, Nierenkarzinom, Ovarialkarzinom, Pankreaskrebs und Prostatakrebs eingesetzt werden.
Hierbei sind insbesondere die Verbindungen
1-(4-Chlorophenyl)-3-(5-hydroxymethylfuran-2-yl)propenon,
3-(5-Hydroxymethylfuran-2-yl-1-(2-hydroxyphenyl)propenon,
3-(4-Hydroxy-3-methoxyphenyl)-1-(2-hydroxyphenyl)propenon und
2-[3-Methoxy-4-(3-methylbut-2-enyloxy)phenyl]chroman-4-on bevorzugt.

Ferner können die erfindungsgemässen Verbindungen als Arzneistoffe gegen bakterielle Infektionen verursacht durch einen Mikroorganismus der Gattung, *Bacillus, Micrococcus, Rhodococcus, Escherichia, Salmonella, Enterococcus, Staphylococcus, Neisseria, Salmonella, Pseudomonas, Treponema, Mycobacterium oder Trichomonas,* insbesondere *Bacillus subtilis, Micrococcus luteus, Rhodococcus equi, Enterococcus faecium, Salmonella choterasuis, Escherichia coli, Candida albicans, Staphylococcus aureus, S. epidermides, S. falcium, Neisseria gonorrhoeae, Salmonella pullorum, Pseudomonas aeruginosa, Treponema pallidum, Bacillus coagulans, Mycobacterium leprae, Trichomonas vaginalis, Bacillus cereus, Bacillus megaterium, Micrococcus roseus, Micrococcus varians, Salmonella typhi, Candida albicans* oder *Mycobacterium tuberculosis* und Pilzinfektionen wie z.B. durch *Candida,* wie *Candida albicans* bei Soor, Pharyngitis, Interdigitalmykosen und gegen lokale und systemische Mykosen eingesetzt werden.

Hierbei sind die Verbindungen
1-(4-Chlorophenyl)-3-(5-hydroxymethylfuran-2-yl)propenon,
3-(4-Hydroxy-3-methoxyphenyl)-1-(2-hydroxyphenyl)propenon,
2-[3-Methoxy-4-(3-methylbut-2-enyloxy)phenyl]chroman-4-on und
2-{1-[2-(3-Methylbut-2-enyloxy)phenyl]ethyliden}malononitril bevorzugt.
Auch können die erfindungsgemässen Verbindungen als Antiseptika eingesetzt werden.

Durch ihre Radikalfängerwirkung sind die erfindungsgemässen Verbindungen, insbesondere 3-(4-Hydroxy-3-methoxyphenyl)-1-(2-hydroxyphenyl)propenon, zur Herstellung von antioxidativen Arzneimittel geeignet.

Zudem können die erfindungsgemässen Verbindungen als zur Herstelllung von immunstimmulierenden bzw. immunmodulierenden Arzneimitteln verwendet werden.

Die vorliegende Erfindung stellt zudem auch Arzneimittel bereit die eine oder mindestens eine erfindungsgemäße Verbindung gegebenenfalls unter Beimischung von im Fachgebiet üblichen Hilfsstoffen und Exzipienten umfassen. Die erfindungsgemäßen Arzneimittel können nach üblichen dem Fachmann bekannten Verfahren und Techniken formuliert/hergestellt werden; wie z.B. in Remington's Remington's Pharmaceutical Sciences,15. Auflage., Mack Publishing Co., New Jersey (1991) beschrieben.
Bevorzugt sind hierbei Darreichungsformen zur oralen, parenteralen (z. B. i.v., s.c., i.p., i.c., intrathekal) und lokalen (z. B. topisch, rectal, vaginal, buccal Applikation im Auge oder durch Inhalation) Applikation.
Somit können die erfindungsgemäßen Arzneimittel insbesondere als Tabletten (insbesondere auch magensaftresistent überzogene Tabletten oder Tabletten mit modifizierter Wirkstofffreigabe), Kapseln (Hart- und Weichgelatinekapseln), Pillen, Granulate, Suppositorien, Ovula, Salben Cremes, Gele, Pflaster, TTS oder auch als Emulsionen, Suspensionen, Lösungen oder rekonstituierbare Pulver (auch zur parenteralen Applikation) vorliegen.

## Patentansprüche

1. Chalkonderivate der allgemeinen Formel III 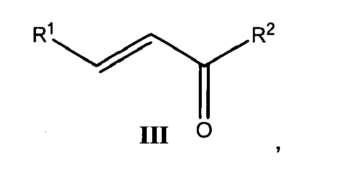 wobei
R¹ einen 5-Hydroxymethylfuran-2-ylrest, einen 4-Methoxyphenylrest, einen 4-Hydroxy-3-methoxyphenylrest, Furan-2-ylrest oder einen 3-Methoxy-4-(3-methylbut-2-enyloxy)phenylrest darstellt und R² einen 4-Chlorphenylrest, einen 2-Hydroxyphenylrest, 2-Hydroxy-5-(3-methylbut-2-enyl)phenylrest, 2,4-Dihydroxyphenylrest, 2,4,6-Trihydroxyphenylrest, 4-Hydroxyphenylrest, 2,2-Dimethyl-2*H*-chromen-6-ylrest, oder einen 2-(3-Methylbut-2-enyloxy)phenylrest darstellt,
mit der Ausnahme von 1-(2-Hydroxyphenyl)-3-(4-methoxyphenyl)propenon und 3-(4-Hydroxy-3-methoxyphenyl)- 1-(2-hydroxyphenyl)propenon.

2. Chalkonderivate gemäss Anspruch 1, ausgewählt aus
1-[2-Hydroxy-5-(3-methylbut-2-enyl)phenyl]-3-(5-hydroxymethylfuran-2-yl)propenon,
1-(2,4-Dihydroxyphenyl)-3-(5-hydroxymethylfuran-2-yl)propenon,
3-(5-Hydroxymethylfuran-2-yl)-1-(2,4,6-trihydroxyphenyl)propenon,
1-(2,2-Dimethyl-2*H*-chromen-6-yl)-3-(4-hydroxy-3-methoxyphenyl)propenon,
1-(2,2-Dimethyl-2*H*-chromen-6-yl)-3-[3-methoxy-4-(3-methylbut-2-enyloxy)phenyl]propenon,
3-Furan-2-yl-1-[2-hydroxy-5-(3-methylbut-2-enyl)phenyl]propenon;
3-(5-Hydroxymethylfuran-2-yl)-1-(4-hydroxyphenyl)propenon,
1-(2,2-dimethyl-2H-chromen-6-yl)-3-(5-hydroxymethylfuran-2-yl)propenon,
1-(2,2-dimethyl-2H-chromen-6-yl)-3-furan-2-ylpropenon,
1-(4-Chlorphenyl)-3-(5-hydroxymethylfuran-2-yl)propenon,
3-(5-Hydroxymethylfuran-2-yl)-1-(2-hydroxyphenyl)propenon,
1-(2-Hydroxyphenyl)-3-[3-methoxy-4-(3-methylbut-2-enyloxy)phenyl]-propenon und
3-[3-Methoxy-4-(3-methylbut-2-enyloxy)phenyl]-1-[2-(3-methylbut-2-enyloxy)phenyl]propenon.

3. Chalkonderivat gemäss Anspruch 1 oder 2, ausgewählt aus
1-(4-Chlorphenyl)-3-(5-hydroxymethylfuran-2-yl)propenon,
3-(5-Hydroxymethylfuran-2-yl)-1-(2-hydroxyphenyl)propenon,
1-(2-Hydroxyphenyl)-3-[3-methoxy-4-(3-methylbut-2-enyloxy)phenyl]-propenon und
3-[3-Methoxy-4-(3-methylbut-2-enyloxy)phenyl]-1-[2-(3-methylbut-2-enyloxy)phenyl]propenon.

4. Chromanderivate der allgemeinen Formel IV 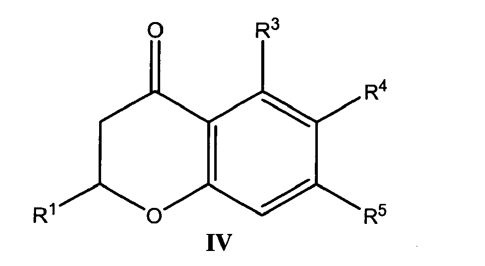 wobei
R¹ einen 5-Hydroxymethylfuran-2-ylrest, einen 4-Methoxyphenylrest, einen 4-Hydroxy-3-methoxyphenylrest, Furan-2-ylrest oder einen 3-Methoxy-4-(3-methylbut-2-enyloxy)phenylrest darstellt und R³, R⁴ und R⁵ unabhängig voneinander ein Wasserstoffatom, einen 5-Hydroxymethylfuran-2-ylrest, einen 4-Methoxyphenylrest, einen 4-Hydroxy-3-methoxyphenylrest oder einen 3-Methoxy-4-(3-methylbut-2-enyloxy)phenylrest darstellen, mit der Massgabe, dass mindestens ein Rest von R³, R⁴ und R⁵ ein Wasserstoffatom ist

5. Chromanderivat gemäss Anspruch 4, wobei
wobei R¹ einen 5-Hydroxymethylfuran-2-ylrest, einen 4-Methoxyphenylrest, einen 4-Hydroxy-3-methoxyphenylrest oder einen 3-Methoxy-4-(3-methylbut-2-enyloxy)phenylrest darstellt,
und R³, R⁴ und R⁵ jeweils ein Wasserstoffatom sind.

6. Chromanderivat gemäss Anspruch 4, ausgewählt aus
2-[3-Methoxy-4-(3-methylbut-2-enyloxy)phenyl]chroman-4-on,
2-(5-Hydroxymethylfuran-2-yl)chroman-4-on,
2-(4-Methoxyphenyl]chroman-4-on und
2-(4-Hydroxy-3-methoxyphenyl)chroman-4-on.

7. Knoevenagelprodukt der allgemeinen Formel **VI** 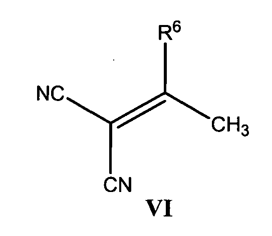 wobei
R⁶ einen 2-(3-Methylbut-2-enyloxy)phenylrest, einen 4-(3-Methylbut-2-enyloxy)phenylrest oder einen 4-Hydroxphenylrest darstellt.

8. Verbindung gemäss Anspruch 7, ausgewählt aus:
2-{1-[2-(3-Methylbut-2-enyloxy)phenyl]ethyliden}malononitril,
2-[1-(4-Hydroxyphenyl)ethyliden]malononitril und
2-{1-[4-(3-Methyl-but-2-enyloxy)phenyl]ethyliden}malononitril.

9. Arzneimittel, welches mindestens eine Verbindung ausgewählt aus einer Verbindung nach einem der Ansprüche 1 bis 8, einschließlich 1-(2-Hydroxyphenyl)-3-(4-methoxyphenyl)propenon und 3-(4-Hydroxy-3-methoxyphenyl)-1-(2-hydroxyphenyl)propenon umfasst.

10. Arzneimittel gemäß Anspruch 9, wobei die Verbindung ausgewählt ist aus
1-(4-Chlorphenyl)-3-(5-hydroxymethylfuran-2-yl)propenon,
3-(5-Hydroxymethylfuran-2-yl)-1-(2-hydroxyphenyl)propenon,
1-(2-Hydroxyphenyl)-3-(4-methoxyphenyl)propenon;
3-(4-Hydroxy-3-methyoxyphenyl)- 1-(2-hydroxyphenyl)propenon,
1-(2-Hydroxyphenyl)-3-[3-methoxy-4-(3-methylbut-2-enyloxy)phenyl]-propenon,
3-[3-Methoxy-4-(3-methylbut-2-enyloxy)phenyl]-1-[2-(3-methylbut-2-enyloxy)phenyl]propenon,
2-[3-Methoxy-4-(3-methylbut-2-enyloxy)phenyl]chroman-4-on,
2-(5-Hydroxymethylfuran-2-yl)chroman-4-on,
2-(4-Methoxyphenyl]chroman-4-on,
2-(4-Hydroxy-3-methoxyphenyl)chroman-4-on,
2-{1-[2-(3-Methylbut-2-enyloxy)phenyl]ethyliden}malononitril,
2-[1-(4-Hydroxyphenyl)ethyliden]malononitril und
2-{1-[4-(3-Methylbut-2-enyloxy)phenyl]ethyliden}malononitril.

11. Verwendung von mindestens einer Verbindung nach einem der Ansprüche 1 bis 8 zur Herstellung eines antiproliferativen, antimikrobiellen (gegen Bakterien und Pilze), antiviralen, immunmodulierenden oder immunstimulierenden Arzneimittels.

12. Verwendung nach Anspruch 11 zur Prophylaxe oder Therapie neoplastischer Erkrankungen, insbesondere Lungenkrebs, Mammakarzinom, Melanoma, Leukämie, Colonkarzinom, Nierenkarzinom, Ovarialkarzinom, Pankreaskrebs und Prostatakrebs.

13. Verwendung nach Anspruch 12, wobei die Verbindung ausgewählt ist aus
1-(4-Chlorphenyl)-3-(5-hydroxymethylfuran-2-yl)propenon (HMF2),
3-(5-Hydroxymethylfuran-2-yl)-1-(2-hydroxyphenyl)propenon (HMF3),
3-(4-Hydroxy-3-methyoxyphenyl)- 1-(2-hydroxyphenyl)propenon (Q20) und
2-[3-Methoxy-4-(3-methylbut-2-enyloxy)phenyl]chroman-4-on (Q25).

14. Verwendung nach Anspruch 11 zur Prophylaxe oder Therapie von Infektionen, verursacht durch einen Mikroorganismus der Gattung *Candida, Bacillus, Micrococcus, Rhodococcus, Escherichia, Salmonella, Enterococcus, Staphylococcus, Neisseria, Salmonella, Pseudomonas, Treponema, Mycobacterium oder Trichomonas.*

15. Verwendung nach Anspruch 14 zur Prophylaxe oder Therapie von Infektionen, verursacht durch *Bacillus subtilis, Micrococcus luteus, Rhodococcus equi, Enterococcus faecium, Salmonella choterasuis, Escherichia coli, Candida albicans, Staphylococcus aureus, S. epidermides, S. falcium, Neisseria gonorrhoeae, Salmonella pullorum, Pseudomonas aeruginosa, Treponema pallidum, Bacillus coagulans, Mycobacterium leprae, Trichomonas vaginalis, Bacillus cereus, Bacillus megaterium, Micrococcus roseus, Micrococcus varians, Salmonella typhi, Candida albicans* oder *Mycobacterium tuberculosis.*

16. Verwendung der Verbindungen 3-(4-Hydroxy-3-methoxyphenyl)- 1-(2-hydroxyphenyl)propenon (Q20) und 1-(2-Hydroxyphenyl)-3-[3-methoxy-4-(3-methylbut-2-enyloxy)phenyl]propenon (Q21) gemäss Anspruch 13 oder 14 gegen *Bacillus subtilis, S. falcium, Salmonella typhi, S. epidermides* und *Candida albicans.*

17. Verwendung der Verbindungen 1-(2-Hydroxyphenyl)-3-(4-methoxyphenyl)-propenon (CH1), 2-{1-[2-(3-Methylbut-2-enyloxy}phenyl]ethyliden}-malononitril (L5), 3-(4-Hydroxy-3-methyoxyphenyl)- 1-(2-hydroxyphenyl)-propenon (Q20), 2-[3-Methoxy-4-(3-methylbut-2-enyloxy)phenyl]chroman-4-on (Q25) und 1-(4-Chlorphenyl)-3-(5-hydroxymethylfuran-2-yl)propenon (HMF2) gemäss Anspruch 14 als Antiseptika.

18. Verfahren zur Herstellung von substituierten Chalkonderivaten der allgemeinen Formel **III:** 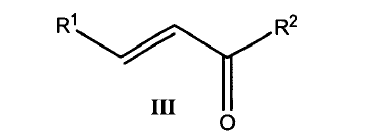 wobei R¹ einen 5-Hydroxymethylfuran-2-ylrest, einen 4-Methoxyphenylrest, einen 4-Hydroxy-3-methoxyphenylrest, Furan-2-ylrest oder einen 3-Methoxy-4-(3-methylbut-2-enyloxy)phenylrest darstellt und R² einen 4-Chlorphenylrest, einen 2-Hydroxyphenylrest, 2-Hydroxy-5-(3-methylbut-2-enyl)phenylrest, 2,4-Dihydroxyphenylrest, 2,4,6-Trihydroxyphenylrest, 4-Hydroxyphenylrest, 2,2-Dimethyl-2*H*-chromen-6-ylrest, oder einen 2-(3-Methylbut-2-enyloxy)phenylrest darstellt, **gekennzeichnet dadurch, dass** substituierte Aldehyde der allgemeinen Formel **I**: 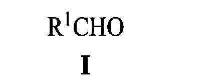 wobei R¹ wie vorstehend definiert ist, in Gegenwart einer Base in einem organischen Lösungsmittel bei einer Reaktionstemperatur im Bereich von 20°C bis 70°C mit einem substituierten Acetophenonderivat der Formel **II:** 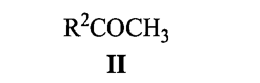 wobei R² wie vorstehend definiert ist, umgesetzt wird, um ein Chalkonderivat der allgemeinen Formel **III** zu erhalten.

19. Verfahren gemäss Anspruch 18, wobei die Base Kaliumhydroxid, Bariumhydroxid oder Piperidin ist.

20. Verfahren gemäss Anspruch 18 oder 19 wobei das organische Lösungsmittel gegebenenfalls wasserfreies Ethanol oder Methanol ist.

21. Verfahren zur Herstellung von Chromanderivaten der allgemeinen Formel **IV** 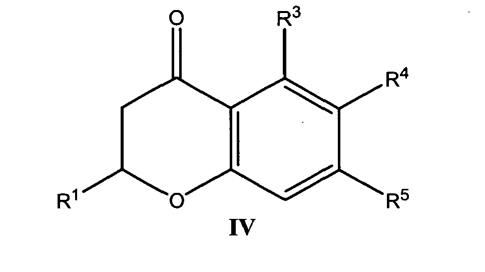 wobei R¹ wie in den Ansprüchen 1 und 18 definiert ist und R³, R⁴ und R⁵ unabhängig voneinander ein Wasserstoffatom, einen 5-Hydroxymethylfuran-2-ylrest, einen 4-Methoxyphenylrest, einen 4-Hydroxy-3-methoxyphenylrest oder einen 3-Methoxy-4-(3-methylbut-2-enyloxy)phenylrest darstellen, mit der Massgabe, dass mindestens ein Rest von R³, R⁴ und R⁵ ein Wasserstoffatom ist, **gekennzeichnet dadurch, dass** ein Chalkon-Derivat der allgemeinen Formel **IIIa** 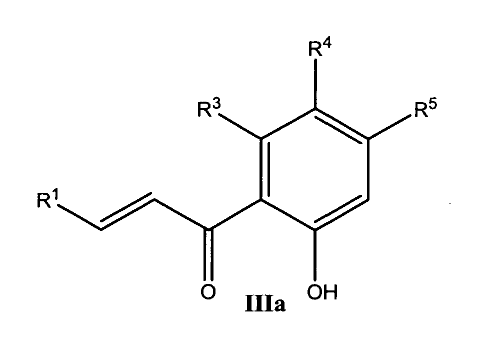 wobei R¹, R³, R⁴ und R⁵ wie vorstehend definiert sind, in Gegenwart einer Base umgesetzt wird, um das substituierte Chroman-4-on-Derivat IV zu erhalten.

22. Verfahren nach Anspruch 21, wobei R¹ einen 5-Hydroxymethylfuran-2-ylrest, einen 4-Methoxyphenylrest, einen 4-Hydroxy-3-methoxyphenylrest oder einen 3-Methoxy-4-(3-methylbut-2-enyloxy)phenylrest darstellt, und R³, R⁴ und R⁵ jeweils ein Wasserstoffatom sind.

23. Verfahren gemäss Anspruch 21 oder 22, wobei die Base Natriumacetat-Trihydrat ist.

24. Verfahren gemäss einem der Ansprüche 21 bis 22, wobei das organische Lösungsmittel Ethanol ist.

25. Verfahren gemäss einem der Ansprüche 21 bis 24, wobei die Umsetzung durch Erwärmen und unter Rückfluss durchgeführt wird.

26. Verfahren zur Herstellung von Knoevenagelprodukten der allgemeinen **Formel VI** 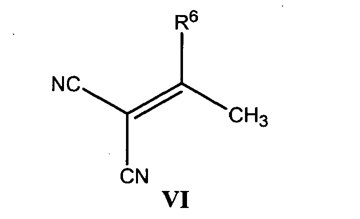 wobei R⁶ einen 2-(3-Methylbut-2-enyloxy)phenylrest, einen 4-(3-Methylbut-2-enyloxy)phenylrest oder einen 4-Hydroxphenylrest darstellt, **gekennzeichnet dadurch, dass** ein Keton der allgemeinen Formel V 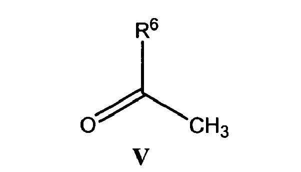 wobei R⁶ wie vorstehend definiert ist, in Gegenwart von Malononitril und einer Mischung aus Ammoniumacetat, Essigsäure und einem organischen Lösungsmittel umgesetzt wird.

27. Verfahren gemäss Anspruch 26, wobei die Umsetzung durch Erwärmen und unter Rückfluss durchgeführt wird.

28. Verfahren gemäss Anspruch 26 oder 27 wobei die Reaktionsdauer 8 bis 10 Stunden beträgt.

29. Verfahren gemäss einem der Ansprüche 26 bis 28, wobei das organische Lösungsmittel Toluol ist.
